Europäisches Patentamt

European Patent Office

Office européen des brevets

(19)

(11) Veröffentlichungsnummer : **0 080 077
B1**

(12) **EUROPÄISCHE PATENTSCHRIFT**

(45) Veröffentlichungstag der Patentschrift :
07.05.86

(51) Int. Cl.⁴ : **C 12 N 11/02**, **C 12 H 1/00,
A 23 L 2/34**

(21) Anmeldenummer : **82110043.5**

(22) Anmeldetag : **30.10.82**

(54) **Trägerfixierte Proteasen und ihre Anwendung in der Biotechnologie.**

(30) Priorität : **19.11.81 DE 3145684**

(43) Veröffentlichungstag der Anmeldung :
**01.06.83 Patentblatt 83/22**

(45) Bekanntmachung des Hinweises auf die Patenterteilung : **07.05.86 Patentblatt 86/19**

(84) Benannte Vertragsstaaten :
**AT BE CH DE FR GB IT LI LU NL SE**

(56) Entgegenhaltungen :
**DE-A- 1 915 970
US-A- 3 597 219**

(73) Patentinhaber : **BOEHRINGER INGELHEIM KG**

**D-6507 Ingelheim am Rhein (DE)**

(72) Erfinder : **Bürger, Erich, Dr.
Leipzigstrasse 7a
D-6530 Bingen (DE)** .
Erfinder : **Hartmeier, Winfried, Prof. Dr.
Melatener Strasse 145a
D-5100 Aachen (DE)**

EP 0 080 077 B1

Jouve, 18, rue St-Denis, 75001 Paris, France

## Beschreibung

Die Erfindung betrifft auf neuartige Weise kovalent an ein Trägerprotein fixierte Proteasen, ihre Herstellung und ihre Anwendung in der Biotechnologie.

Proteasen haben erhebliche Bedeutung in der Getränkeindustrie, da bei vielen klaren Getränken (wie Wein, Bier und Fruchtsäfte) in der Wärme oder auch beim Abkühlen Eiweißtrübungen auftreten können.

Diesem Problem wird z. T. bis heute durch Bentonit-Behandlung begegnet. Hierdurch wird zwar die erforderliche Stabilität erreicht, jedoch tritt gleichzeitig eine Reihe gravierender Nachteile auf. So wird durch die starke Adsorptionswirkung auch eine Reihe wertvoller Substanzen, wie Aroma-, Bukett- und/oder Farbstoffe entfernt, ferner können unerwünschte Metallionen eingetragen werden, und drittens ergeben sich erhebliche Mengen an Trub, dessen Aufarbeitung einigen Aufwand erfordert.

Es wurde daher bereits vorgeschlagen, die den Wärme- bzw. Kältetrub bildenden Proteine durch Proteasen aufzulösen (s. Z. für Lebensmitteluntersuchung und -forschung 134 (1967), S. 87-97).

Jedoch ergeben sich auch bei dieser Methode Nachteile. Zunächst sind die Kosten für die Enzymanreicherung und -isolierung recht hoch, da die Enzyme nicht stabil sind und auch nicht rückgewonnen werden können. Ferner ist das Verbleiben von löslichen Enzymen im Getränk lebensmittelrechtlich bedenklich oder sogar unzulässig.

Zur Lösung dieser Probleme bietet sich an, die verwendeten Enzyme unlöslich zu machen, indem man sie an wasserunlösliche Trägerstoffe fixiert. Dies erlaubt, die Enzyme zurückzugewinnen und mehrfach einzusetzen.

Methoden zur Enzymfixierung sind bereits mannigfach bekannt. Eine Übersicht über die neuesten Ergebnisse auf diesem Gebiet gibt z. B. J.C. Johnson (Herausgeber) in « Immobilized Enzymes, Preparation and Engineering » ; Noyes Data Corp., Park Ridge, N.J., USA (1979).

Durch die Trägerfixierung der Enzyme können, neben den oben angegebenen, weitere Vorteile erreicht werden. Dies sind z. B.

kontinuierliche Prozeßführung ;
erhöhte Stabilität ;
kleineres Reaktionsvolumen ;
keine Verunreinigung der Reaktionsansätze ;
keine Hitzefällung des Enzyms.

Zur Bindung eines Enzyms an eine unlösliche Trägersubstanz gibt es kein Standardverfahren. Das Ziel, die biologische Aktivität des Enzyms möglichst weitgehend zu erhalten, erfordert eine individuelle Anpassung von Trägersubstanz und Immobilisierungsmethode an das Enzym.

In der DE-A-19 15 970 wird ein Verfahren zur Herstellung von trägerfixierten Proteinsubstanzen beschrieben, bei dem man einen Träger mit einer Lösung einer aktiven Proteinsubstanz in Gegenwart eines Brückenbildners in Berührung bringt und anschließend den Träger, auf dem die aktive Proteinsubstanz fixiert ist, isoliert.

Besonders beliebte (da einfach zu verarbeitende und kostengünstige) Trägersubstanzen für diesen Zweck sind gelbildende Proteine wie Gelatine oder frisches Eiklar (s. hierzu beispielsweise die DE-AS 22 46 002). Diese Trägersubstanzen eignen sich jedoch nicht für die Fixierung von Enzymen mit proteolytischer Aktivität, da sie von ihnen hydrolysiert werden.

Es wurde daher bereits vorgeschlagen (s. DE-AS 26 36 206.1-41), die gelbildenden Proteine durch chemische Härtung (z. B. Formaldehyd, Glutardialdehyd oder Diisocyanat) zu denaturieren, um sie dadurch unangreifbar für die auf ihnen fixierten Proteasen zu machen. Auf diese Weise gelingt beispielsweise die Trägerfixierung des Labenzyms oder auch von Pepsin (s. Biotechnology Letters 1 (1979) S. 225-230).

Überraschenderweise wurde nun gefunden, daß Proteasen auch dann an gelbildende Proteine fixiert werden können, wenn man eine aufwendige chemische Vorbehandlung der Proteine unterläßt.

Gegenstand der Erfindung ist daher ein Verfahren zur Immobilisierung von Proteasen an gelbildenden Proteinen, das dadurch gekennzeichnet ist, daß man die Protease mit einem gelbildenden Protein (z. B. Gelatine, Eiklar, Sojaprotein u. a., vorzugsweise Albumin) versetzt, das Gemisch mit einem mit Wasser mischbaren organischen Lösungsmittel (Fällungsmittel) behandelt und anschließend die entstandene Teilchensuspension mit bi- und/oder polyfunktionellen Vernetzungsmitteln umsetzt. Gegenstand der Erfindung sind ferner die so erhaltenen trägerfixierten Enzympräparate und ihre Verwendung zum Abbau von Proteinen.

Das Gemisch aus dem proteolytischen Enzym und dem gelbildenden Protein kann durch Auflösen oder Suspendieren des Enzyms in einer wässrigen Lösung des gelbildenden Proteins hergestellt werden. Dabei wird die Temperatur vorzugsweise so eingestellt, daß das aktive Gemisch in flüssiger Form erhalten wird.

Das Gemisch aus proteolytischem Enzym und gelbildendem Protein wird vorzugsweise so hergestellt, daß man zunächst das proteolytische Enzym (z. B. Pepsin, Papain etc.) in Wasser löst und es dann mit einer wässrigen Lösung des gelbildenden Proteins vermischt. Dieses Gemisch wird dann mit einem organischen Lösungsmittel, das enzymfällend wirkt und mit dem Enzym verträglich ist, versetzt. Beispiele dafür sind Aceton, Äthanol, Isopropanol oder Butanol. Die durch das Fällungsmittel entstandene Teilchensuspension wird derart und solange mit bifunktionellen oder polyfunktionellen Vernetzungsmitteln reagieren gelassen bis eine möglichst vollständige Vernetzung des Gemisches aus dem proteolytischen Enzym und dem gelbildenden Protein erreicht ist. Es ist auch möglich, das teilchenförmige Gemisch vorher von der organischen Flüssigkeit abzutrennen und erst dann mit dem Vernetzungsmittel zu behandeln.

Für die Vernetzungsreaktion kommen bifunktionelle und polyfunktionelle Vernetzungsmittel für Proteine in Frage. Vorzugsweise wird eine wässrige Lösung von Glutardialdehyd verwendet. Es ist auch möglich, das Kopplungsreagenz in einer der obengenannten enzymfällend wirkenden Flüssigkeiten zu lösen und in dieser Form dem wässrigen Gemisch aus proteolytischem Enzym und gelbildenden Protein zuzusetzen.

Dieses Reaktionsgemisch aus proteolytischem Enzym, gelbildendem Protein, enzymfällender Flüssigkeit und dem Vernetzungsmittel wird einige Zeit, vorzugsweise 0,5-4 Stunden kräftig gerührt. Die Kopplungsreaktion wird üblicherweise bei Raumtemperatur durchgeführt.

Nach Beendigung der Bindungsreaktion wird das Präparat mehrfach, vorzugsweise mit Wasser, gewaschen. Das so erhaltene Produkt kann dann auf den gewünschten Wassergehalt getrocknet werden (z. B. durch Sprühtrocknung oder Acetontrocknung).

Die nach der oben dargestellten Arbeitsweise hergestellten Enzymprodukte können in ansatzweise durchgeführten Verfahren zur Proteolyse von Eiweiß (Eiweißabbau) unter Abtrennung und erneuter Verwendung des Enzymproduktes oder in kontinuierlichen Verfahren in einem Enzymreaktor angewandt werden. Sie eignen sich besonders zur Behandlung proteinhaltiger Getränke (wie z. B. Wein, Bier und Fruchtsäfte), bei denen keine Enzyme im Endprodukt verbleiben sollen.

Bei dem verwendeten proteolytischen Enzym handelt es sich vorzugweise um Pepsin.

Die Bestimmung der Pepsin-Aktivität der löslichen sowie der immobilisierten Form, erfolgt nach der Methode von Anson

(Rick, W & Fritsch, W.-P. in Bergmeyer (Herausgeber) : Methoden der enzymatischen Analyse, 3. Auflage., Band 1, S. 1086-1090, Verlag Chemie, Weinheim, 1974).

Die folgenden Beispiele dienen zur weiteren Erläuterung der Erfindung :

Beispiel 1

2 kg Pepsin wurden bei Zimmertemperatur in 10 l dest. Wasser gelöst. Diese Enzymlösung wurde mit einer wässrigen Albuminlösung (200 g Albumin in 5 l dest. Wasser) vermischt. Die so erhaltene Suspension wurde unter Rühren und bei einer Temperatur von 25 °C in 24 l Isopropanol (80 %) gegeben und anschließend mit 1 l Glutardialdehyd (25 %) versetzt. Dieses Reaktionsgemisch wurde 2 Stunden bei 25 °C gerührt. Nach Ablauf dieser Zeit wurde das Reaktionsprodukt abzentrifugiert und gründlich mit 200 l Wasser gewachen. Das gewaschene Produkt wurde über einen Laborsprühtrockner mit Zweistoffdüse bei einer Lufteingangstemperatur von 155 °C, einer Luftausgangstemperatur von 75 °C und einem Luft durchsatz von 420 Nm³/h getrocknet. Das verwendete Pepsin (20 kg) hatte eine Gesamt-Ausgangsaktivität von 6 000 AE (Anson-Einheiten). Das trägerfixierte Trockenpräparat betrug 415 g, und zeigte eine Gesamt-Aktivität von 3 800 AE, was einer Wiedergewinnung nach der Immobilisierung und Sprühtrocknung von 63,3 % entspricht.

Beispiel 2

20 g Pepsin aus Schweinemagen (5 AE/g) wurden mit 2 g Ei-Albumin in 150 ml dest. Wasser gelöst. Zu dieser Lösung wurden 200 ml eiskaltes Aceton hinzugerührt. Nach einigen Minuten wurde zu dieser Suspension 10 ml 25 %-iger Glutardialdehyd-Lösung zugesetzt und bei Zimmertemperatur gerührt oder geschüttelt. Nach einer Reaktionszeit von ca. 2 Stunden wurde das Reaktionsprodukt abfiltriert und gründlich mit dest. Wasser gewaschen. Der Rückstand wurde in eiskaltem Aceton suspendiert. Durch Filtration wurde das immobilisierte Enzymprodukt vom Aceton getrennt, der Rückstand anschließend bei 45 °C im Vakuum-Trockenschrank getrocknet. Es resultierten 2,5 g trägerfixiertes Trockenpräparat mit einer Aktivität von 11 AE/g.

**Patentansprüche**

1. Verfahren zur Herstellung von auf gelbildenden Proteinen trägerfixierten Proteasen, dadurch gekennzeichnet, daß man die Proteasen mit einem gelbildenden Protein versetzt, das Gemisch mit einem enzymfällend wirkenden organischen Lösungsmittel behandelt und anschließend die entstandene Teilchensuspension mit einem oder mehreren Vernetzungsmitteln umsetzt.

2. Verfahren nach Anspruch 1, dadurch gekennzeichnet, daß man als gelbildendes Protein Albumin einsetzt.

3. Verfahren nach einem der Ansprüche 1 oder 2, dadurch gekennzeichnet, daß es sich bei der Protease um Pepsin handelt.

4. Verfahren nach einem der vorhergehenden Ansprüche, dadurch gekennzeichnet, daß als Vernetzungsmittel Glutardialdehyd eingesetzt wird.

5. Trägerfixiertes Proteasepräparat herstellbar nach einem der vorhergehenden Ansprüche.

6. Proteasepräparat nach Anspruch 5, dadurch gekennzeichnet, daß Pepsin kovalent mittels Glutardialdehyd an ein gelbildendes Protein gebunden ist.

7. Proteasepräparat nach Anspruch 6, dadurch gekennzeichnet, daß das gelbildende Protein Albumin ist.

8. Verwendung eines Proteasepräparats nach einem der Ansprüche 5 bis 7 in biotechnologischen Verfahren.

9. Verwendung eines Proteasepräparats nach einem der Ansprüche 5 bis 7 zur Klärung von Getränken.

10. Verwendung eines Proteasepräparats nach Anspruch 7 zur Beseitigung des Eiweißtrubs in Wein oder Bier.

## Claims

1. Process for the preparation of proteases fixed on a carrier of gel-forming proteins, characterised in that the proteases are mixed with a gel-forming protein, the mixture is treated with an organic solvent having an enzyme-precipitating activity and subsequently the particle suspension formed is reacted with one or more cross-linking agents.

2. Process as claimed in claim 1, characterised in that albumin is used as the gel-forming protein.

3. Process as claimed in one of claims 1 or 2, characterised in that the protease is pepsin.

4. Process as claimed in one of the preceding claims, characterised in that glutardialdehyde is used as the cross-linking agent.

5. Protease preparation fixed on a carrier, capable of being prepared according to one of the preceding claims.

6. Protease preparation as claimed in claim 5, characterised in that pepsin is covalently bonded by means of glutardialdehyde to a gel-forming protein.

7. Protease preparation as claimed in claim 6, characterised in that the gel-forming protein is albumin.

8. Use of a protease preparation as claimed in one of claims 5 to 7 in biotechnological processes.

9. Use of a protease preparation as claimed in one of claims 5 to 7 for clarifying drinks.

10. Use of a protease preparation as claimed in claim 7 for removing protein turbidity from wine or beer.

## Revendications

1. Procédé de préparation de protéases fixées sur support sur des protéines formant des gels, caractérisé en ce qu'on additionne les protéases d'une protéine formant un gel, en ce qu'on traite le mélange par un solvant organique ayant une action de précipitation des enzymes, puis en ce qu'on fait réagir la suspension de particules formée avec un ou plusieurs agents de réticulation.

2. Procédé suivant la revendication 1, caractérisé en ce qu'on utilise, comme protéine formant un gel, de l'albumine.

3. Procédé suivant l'une des revendications 1 ou 2, caractérisé en ce que la protéase est la pepsine.

4. Procédé suivant l'une des revendications précédentes, caractérisé en ce qu'on utilise comme agent de réticulation du glutardialdéhyde.

5. Préparation de protéase fixée sur support, pouvant être préparée suivant l'une des revendications précédentes.

6. Préparation de protéase suivant la revendication 5, caractérisé en ce que la pepsine est liée par covalence au moyen de glutardialdéhyde sur une protéine formant un gel.

7. Préparation de protéase suivant la revendication 6, caractérisé en ce que la protéine formant un gel est l'albumine.

8. Utilisation d'une préparation de protéase suivant l'une des revendications 5 à 7 dans des procédés biotechnologiques.

9. Utilisation d'une préparation de protéase suivant l'une des revendications 5 à 7 pour la clarification de boissons.

10. Utilisation d'une préparation de protéase suivant la revendication 7 pour éliminer le trouble protéinique dans le vin ou la bière.